# EUROPEAN PATENT APPLICATION

(11) **EP 3 718 491 A2**
(43) Date of publication of application: **07.10.2020**
(21) Application number: 20165969.5
(22) Date of filing: 26.03.2020
(51) Int. Cl.: A61B 17/12

(54) **ANEURYSM TREATMENT DEVICE**

(30) Priority: 27.03.2019 US 201916366115
(71) Applicant: DePuy Synthes Products, Inc., Raynham MA 02767 (US)
(72) Inventor: SOTO DEL VALLE, Ariel, RAYNHAM, Delaware 02767 (US); GOROCHOW, Lacey, RAYNHAM, Delaware 02767 (US); LORENZO, Juan, RAYNHAM, Delaware 02767 (US)
(74) Representative: Small, Gary James

(57) **Abstract**

An implant having an embolic portion and a braided tubular occluding portion can be delivered through a catheter and implanted in an aneurysm. The embolic portion can be an elongated embolic structure that can coil as it is inserted into an aneurysm sac. The occluding portion can be a braided tube that is shaped to encircle the elongated embolic portion during delivery and expands to form a cup shape within the aneurysm sac, nesting in the aneurysm neck when implanted. The implant can include a closure mechanism positioned between the occluding portion and the embolic portion that can serve the purpose of restricting an end of the occluding portion at the trough of the cup shape, connecting the embolic portion to the occluding portion, and/or providing a detachment feature for connecting to a delivery system.

## Description

### Field of Invention

The present invention generally relates to medical instruments, and more particularly, to embolic implants for aneurysm therapy.

### Background

Cranial aneurysms can be complicated and difficult to treat due to their proximity to critical brain tissues. Prior solutions have included endovascular treatment whereby an internal volume of the aneurysm sac is removed or excluded from arterial blood pressure and flow. Current alternatives to endovascular or other surgical approaches can include intravascularly delivered treatment devices that either fill the sac of the aneurysm with embolic material or block the entrance or neck of the aneurysm. Both approaches attempt to prevent blood flow into the aneurysm. When filling an aneurysm sac, the embolic material clots the blood, creating a thrombotic mass within the aneurysm. When treating the aneurysm neck, blood flow into the entrance of the aneurysm is inhibited, inducing venous stasis in the aneurysm and facilitating a natural formation of a thrombotic mass within the aneurysm.

Current intravascularly delivered devices typically utilize multiple embolic coils to either fill the sac or treat the entrance. Naturally formed thrombotic masses formed by treating the entrance of the aneurysm with embolic coils can improve healing compared to aneurysm masses packed with embolic coils by reducing possible distention from arterial walls and permitting reintegration into the original parent vessel shape along the neck plane. However, embolic coils delivered to the neck of the aneurysm can potentially have the adverse effect of impeding the flow of blood in the adjoining blood vessel; at the same time, if the entrance is insufficiently packed, blood flow can persist into the aneurysm. Treating certain aneurysm morphology (e.g. wide neck, bifurcation, etc.) can required ancillary devices such a stents or balloons to support the coil mass and obtain the desired packing density. Once implanted, the coils cannot easily be retracted or repositioned. Furthermore, embolic coils do not always effectively treat aneurysms as aneurysms treated with multiple coils often recanalize or compact because of poor coiling, lack of coverage across the aneurysm neck, because of flow, or large aneurysm size.

Alternatives to embolic coils are being explored, for example a tubular braided implant is disclosed in US Patent Publication Number 20180242979, incorporated herein by reference. Tubular braided implants have the potential to easily, accurately, and safely treat an aneurysm or other arterio-venous malformation in a parent vessel without blocking flow into perforator vessels communicating with the parent vessel. Compared to embolic coils, however, tubular braided implants are a newer technology, and there is therefore capacity for improved geometries, configurations, delivery systems, etc. for the tubular braided implants.

There is therefore a need for improved methods, devices, and systems for implants for aneurysm treatment.

### Summary

It is an object of the present invention to provide systems, devices, and methods to meet the above-stated needs. Generally, in examples herein, an implant having an expandable occluding portion and a coiling embolic portion can be delivered through a catheter and implanted in an aneurysm. The embolic portion can be an elongated embolic structure that can coil as it is inserted into an aneurysm sac. The occluding portion can be a braided tube that encircles the elongated embolic portion during delivery and expands to form a cup shape within the aneurysm sac, nesting in the aneurysm neck when implanted. The implant can include a closure mechanism positioned between the occluding portion and the embolic portion that can serve the purpose of restricting an end of the occluding portion at the trough of the cup shape, connecting the embolic portion to the occluding portion, and/or providing a detachment feature for connecting to a delivery system.

An example implant can have an embolic portion, an occluding portion, and a closure mechanism defining a boundary between the embolic portion and the occluding portion. The implant can be movable from a delivery configuration to an implanted configuration. In the delivery configuration, the implant can be sized to be delivered to a treatment site through a lumen of a catheter as follows: the embolic portion can extend proximally from a distal end of the implant to the closure mechanism, the occluding portion can extend proximally from the closure mechanism, the occluding portion can have a fold near a proximal end of the implant, the occluding portion can extend distally to encompass the closure mechanism and at least some of the embolic portion, and the occluding portion can have an open end that encircles the embolic portion. In the implanted configuration the implant can be sized to secure within an aneurysm as follows: the embolic portion can be sized to wind within and fill a majority of an aneurysm sac and the occluding portion can be sized to contact an aneurysm wall and partially or completely occlude an aneurysm neck.

The embolic portion and the occluding portion can both be a tubular braided structure. Alternatively, the embolic portion can be an embolic coil and the occluding portion can be a tubular braid.

In the implanted configuration, the occluding portion can form a cup shape. An open end of the occluding portion can define a ridge of the cup shape and the closure mechanism can be positioned at a trough of the cup shape. In the implanted configuration, the occluding portion can be a braided mesh having pores sized to inhibit blood flow into the aneurysm. In the implanted configuration, the occluding portion can anchor to the aneurysm wall and inhibit the embolic portion from exiting the aneurysm sac. In the implanted configuration, the embolic portion can have a substantially uniform circumference along a majority of its length.

An example implant suitable for endovascular treatment of an aneurysm in a patient can include a substantially tubular structure, an elongated embolic member, and a band.

The tubular structure can have a constrained proximal end region and an open distal end region. The tubular structure can be movable from a collapsed condition to an expanded condition. In the collapsed condition, the tubular structure can be sized for insertion through vasculature of the patient and through a neck of the aneurysm. In the expanded condition, the tubular structure can be shaped so that its open distal end region has a circumference sufficiently large to contact an interior wall of the aneurysm and its constrained proximal end region is positioned to converge near a center of the aneurysm neck. In the expanded condition, the tubular structure can have a ridge defined by the open distal end region and a trough centered around the constrained proximal end region. The tubular structure can be a braided mesh, and, in the expanded condition, the braided mesh can have pores sized to inhibit blood flow into the aneurysm. In the expanded condition, the tubular structure can be sized to anchor to the aneurysm wall and inhibit the elongated embolic member from exiting the aneurysm sac.

The elongated embolic member can be attached to the constrained proximal end region of the tubular structure and extend distally therefrom. The elongated embolic member can be positioned to be encircled by the tubular structure when the tubular structure is in the collapsed condition. The elongated member can be bendable to loop within an aneurysm sac.

The band can be disposed on the constrained proximal end region of the tubular structure. The band can inhibit expansion of the constrained proximal end region.

The elongated embolic member and the tubular structure can both be tubular braided structures. Alternatively, the elongated embolic member can be an embolic coil and the tubular structure can be braided.

A method of treating an aneurysm can include one or more of the following steps presented in no particular order, and the method can include additional steps not included here. An implant having an elongated embolic portion, an expandable occluding portion, and a closure mechanism can be provided. The closure mechanism can be joined to an end of the elongated embolic portion and joined to an end of the expandable occluding portion (referred to hereafter as the "constrained end"). The expandable occluding portion can be inverted such that the expandable occluding portion envelopes the closure mechanism and a portion of the elongated embolic portion. The implant can be translated distally through a catheter configured as follows: a free end of the elongated embolic portion can be positioned at a distal end of the implant, the constrained end of the expandable occluding portion can be positioned at a proximal end of the implant, and the open end of the expandable occluding portion can be positioned distal the constrained end. The elongated embolic portion can be inserted into a sac of the aneurysm such that the elongated embolic portion loops within the sac. The open end of the expandable occluding portion can expand within the sac near an aneurysm neck. The expandable occluding portion can contact an aneurysm wall. The closure mechanism can be positioned at a center of the aneurysm neck.

The elongated embolic portion and the expandable occluding portion can be formed from a contiguous tubular braid. Alternatively, the embolic portion can be formed from an embolic coil and the expandable portion can be formed from a tubular braid. The expandable occluding portion can be expanded into a bowl shape having a ridge and a trough, the ridge defined by the open end of the expandable occluding portion and sized to be positioned near the wall of the aneurysm, and the trough centered around the closure mechanism. The expandable occluding portion can be formed from a braided mesh, and pores of the expanded braided mesh can be sized to inhibit blood flow into the aneurysm. The expandable occluding portion can be anchored to the aneurysm wall. The elongated embolic portion can be inhibited from exiting the aneurysm sac. The process of anchoring the expandable occluding portion to the aneurysm wall can include providing a force from the elongated embolic portion against an interior surface of the expandable occluding portion to press an exterior surface of the expandable occluding portion to the aneurysm wall.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1A is an illustration of an example implant in a collapsed or delivery configuration according to aspects of the present invention;
FIG. 1B is an illustration of the example implant of FIG. 1A in an implanted or expanded configuration according to aspects of the present invention;
FIGs. 2A through 2E are illustrations of an example implant during implantation steps according to aspects of the present invention;
FIG. 3A is an illustration of an example implant in a collapsed or delivery configuration according to aspects of the present invention;
FIG. 3B is an illustration of the example implant of FIG. 3A in an implanted or expanded configuration according to aspects of the present invention; and
FIG. 4 is a flow diagram outlining example method steps for treating an aneurysm according to aspects of the present invention.

### Detailed Description

As object of the present invention is to provide an embolic implant suitable for endovascular treatment of an aneurysm in a patient. The implant can have two parts that can reshape upon delivery to a treatment site. A first, elongated part can extend from the distal end of the implant during delivery and can reshape into a complex coiled structure within an aneurysm sac upon delivery. A second, tubular braided part can be positioned at a proximal end of the implant, be folded over the first, elongated part during delivery, and can expand to form a cup shape that nests within the aneurysm at the aneurysm neck when implanted. The expanded braided cup can hold the looped elongated part in the aneurysm sac. The expanded cup can cover the neck of the aneurysm and provide flow diversion properties from the pores of the braid.

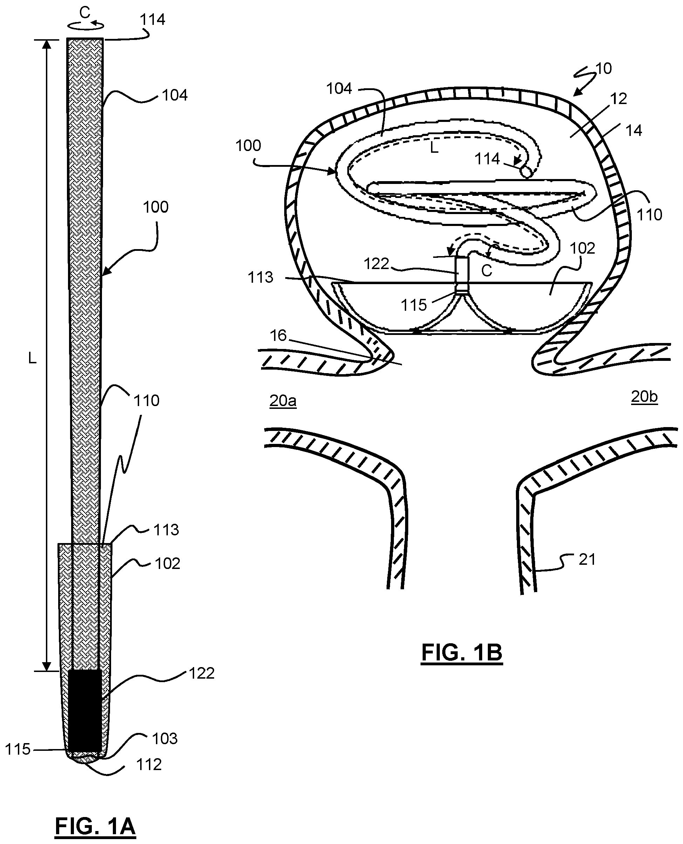

FIG. 1A illustrates an example implant 100 in a collapsed or delivery configuration. In the delivery configuration, the implant 100 can be sized to be delivered to a treatment site through a catheter inserted in vasculature of a patient. FIG. 1B illustrates the example implant 100 illustrated in FIG. 1A in an expanded or implanted configuration. In the implanted configuration, the implant 100 can be positioned in a sac 12 of an aneurysm 10 to divert blood flow from a neck 16 of the aneurysm 10 and fill the sac 12 with embolic material. The embolic material in the aneurysm sac 12 can promote the formation of a thrombotic mass in the aneurysm 10, and the diversion of blood flow from the aneurysm neck 16 can induce venous stasis in the aneurysm and reduce the likelihood that the aneurysm recanalizes after an aneurysm treatment procedure.

Referring collectively to FIGs. 1A and 1B, the implant 100 can include an elongated portion 104, an occluding portion 102, and a closure mechanism 122. The elongated portion 104 and the occluding portion 102 can each be braided. The occluding portion 102 and elongated portion 104 can be manufactured as two separate braided structures or as a contiguous braided structure. Braided portions can be made from braided Nitinol, cobalt, chromium, plastic, or other suitable material. Portions of the implant 100 can be made from a memory shape material having a predetermined shape and a deformed shape. The memory shape material can be in the deformed shape when the implant 100 is in the delivery configuration and can move toward the predetermined shape when the implant 100 is in the expanded or implanted configuration. When implanted, the memory shape material can be restricted by anatomical geometries and the memory shape material can take a shape that approaches the predetermined shape but does not match the predetermined shape due to the anatomical restrictions.

The closure mechanism 122 can be joined to the elongated portion 104 and the occluding portion 102 and can define a boundary between the elongated portion 104 and occluding portion 102. The closure mechanism 122 can be joined at an end of each of the elongated portion 104 and the occluding portion 102 to connect the elongated portion 104 to the occluding portion 102 if the two parts 102, 104 are made from separately manufactured braided sections. The closure mechanism 122 can include band, and/or a mechanism for detachably attaching to a delivery system.

Referring to FIG. 1A, in the delivery configuration, the closure mechanism 122 can be positioned at the proximal end 112 of the implant 100, the elongated portion 104 can extend distally from the closure mechanism 122 to a distal end 114 of the implant 100, and the occluding portion 102 can extend proximally from the closure mechanism 122 and include a fold 103 at the proximal end 112 of the implant 100. In the delivery configuration, the occluding portion 102 can extend distally from the fold 103 to wrap around the closure mechanism 122 and part of the length L of the elongated portion 104, and the occluding portion 102 can have an open end 113 that circles the elongated portion 104.

To achieve this folded structure, the occluding portion 102 can first be connected to the closure mechanism so that it extends proximally from the closure mechanism 122, the open end 113 being positioned in the proximal direction from the closure mechanism 122, and the occluding portion 102 can then be inverted and moved distally to envelope the closure mechanism 122 and part of the elongated portion 104, thereby forming the fold 103 at the proximal end 112 of the implant 100 and moving the open end 113 to encircle the elongated portion 104. In the delivery configuration, the closure mechanism 122 can include a detachment feature detachably attached to a delivery system.

Referring to FIG. 1B, in the implanted configuration, the occluding portion 102 can extend across the aneurysm neck 16, the elongated portion 104 can wind within the aneurysm sac 12, and the closure mechanism 122 can be positioned near the center of the aneurysm neck 16. In the implanted configuration, the occluding portion 102 can extend from the closure mechanism 122 to engage a wall 14 and anchor within the aneurysm sac 12. By anchoring within the sac 12, the occluding portion 102 can maintain proper positioning of the implant 100 in the aneurysm 10 and prevent portions of the implant 100, such as the elongated portion 104, from extending into a blood vessel 20a, 20b, 21 or exiting the aneurysm sac 12.

The implanted occluding portion 102 can form a cup shape, and the elongated portion 104 can wind or loop around in a complex coiled shape within the cupped occluding portion 102 and within the aneurysm sac 12. The open end 113 of the occluding portion 102 can define a ridge of the cupped shape, and the closure mechanism 122 can be positioned centrally within the cupped shape, or near the trough. The closure mechanism 122 can constrain an end 115 of the occluding portion 102 to a completely closed size or a small enough size to inhibit blood flow from entering the aneurysm neck 16. The looped elongated portion 104 can press against the cupped occluding portion 102 and provide a force against an interior surface of the occluding portion 102 to press an exterior surface of the occluding portion 102 to the aneurysm wall 14 to further secure the anchoring of the cupped occluding portion 102 within the aneurysm sac 12.

The looped elongated portion 104 can fill a majority of the aneurysm sac 12. The implanted occluding portion 102 can be a braided mesh with a porosity sized to inhibit blood flow into the aneurysm 10. The elongated portion can have a substantially uniform circumference C along much or all of its length L, and it can maintain the substantially uniform circumference C as it moves from the delivery configuration to the implanted configuration.

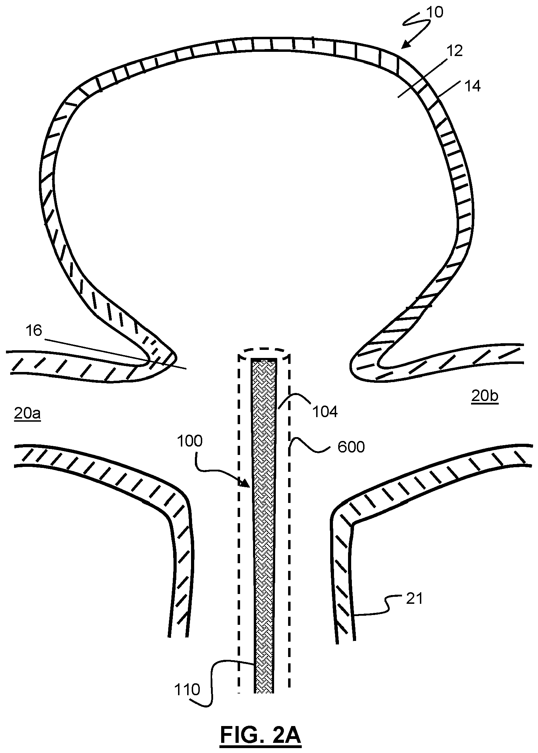

FIGs. 2A through 2E are illustrations of an example implant during implantation steps. FIG. 2A illustrates an implant 100 positioned within a catheter 600 near an aneurysm 10. The aneurysm 10 is illustrated positioned at a bifurcated blood vessel having a stem vessel 21, a first side branch vessel 20a, and a second side blood vessel 20b. The catheter 600 can approach the aneurysm 10 from the stem vessel 21. It is contemplated that example implants 100 disclosed herein can be used for treating sidewall vessels according to methods described herein and as would be understood by a person of ordinary skill in the art. At the instant illustrated in FIG. 2A, the implant 100 is collapsed in the catheter 600. The implant 100 can include memory shape material that is in a deformed shape while it is collapsed in the catheter 600.

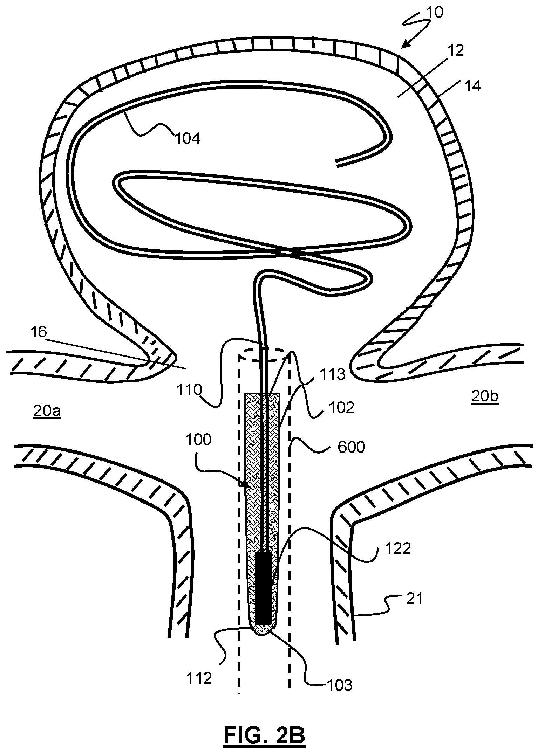

FIG. 2B illustrates the implant 100 during implantation. The implant 100 can include an occluding portion 102 and an elongated embolic portion 104. As the implant is translated distally through the catheter, a free end of the elongated embolic portion 104 can be positioned at a distal end 114 of the implant 100, the constrained end 115 of the expandable occluding portion 102 can be positioned at a proximal end 112 of the implant 100, and the open end 113 of the expandable occluding portion 102 can be positioned distal the constrained end 115. Configured thusly, the embolic portion 104 can be oriented in the catheter 600 so that when the implant 100 is pushed distally out of the catheter 600, a majority of the embolic portion 104 is pushed out of the catheter 600 and into the sac 12 before the occluding portion 102 begins to exit the catheter 600.

The embolic portion 104 can maintain its circumference C as it exits the catheter 600. The embolic portion 104 can wind or loop within the aneurysm sac 12 in response to contacting the aneurysm wall 14. Additionally, or alternatively, the embolic portion 104 can include memory shape material having a predetermined shape and a deformed shape. The predetermined shape can be a complex looped shape, and the deformed shape can be substantially straight. The embolic portion 104 can wind or loop within the aneurysm sac 12 in response to the memory shape material moving from the deformed shape toward the predetermined shape as the embolic portion 104 contacts blood as it exits the catheter 600. Additionally, or alternatively, the embolic portion 104 can include a flexible elastically deformable material having a relaxed shape that is a looped shape. The flexible elastically deformable material can be uncoiled to a substantially straight strand during delivery through a catheter and can collapse into the looped shape upon exiting the catheter 12.

The closure mechanism 122 can include a detachment feature for detachably attaching to a delivery system. At the instant illustrated in FIG. 2B, the detachment feature can be attached to the delivery system. While the detachment feature is attached to the delivery system, all or part of the implant 100 can be withdrawn proximally into the catheter and extracted from the patient or repositioned and re-implanted.

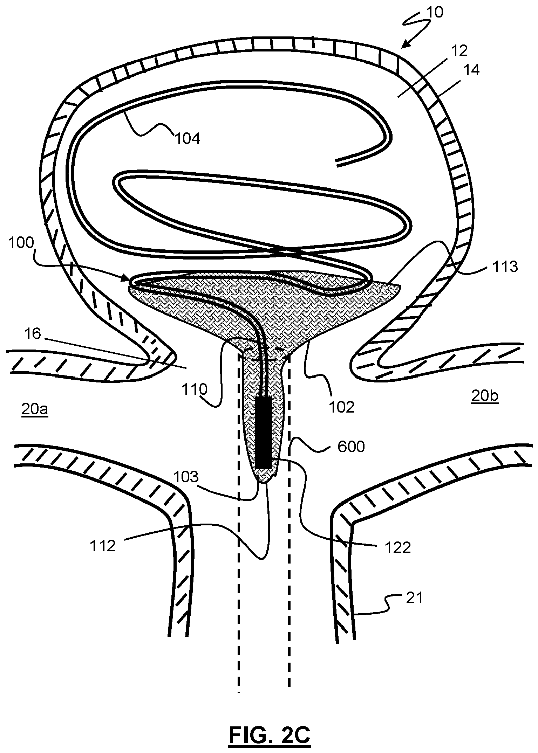

FIG. 2C illustrates the implant 100 during implantation as the occluding portion 102 begins to exit the catheter 600 and expand. The open end 113 of the expandable occluding portion 102 can be expanded within the sac 12 near the neck 16 of the aneurysm. At the instant illustrated in FIG. 2C, the detachment feature can remain attached to the delivery system.

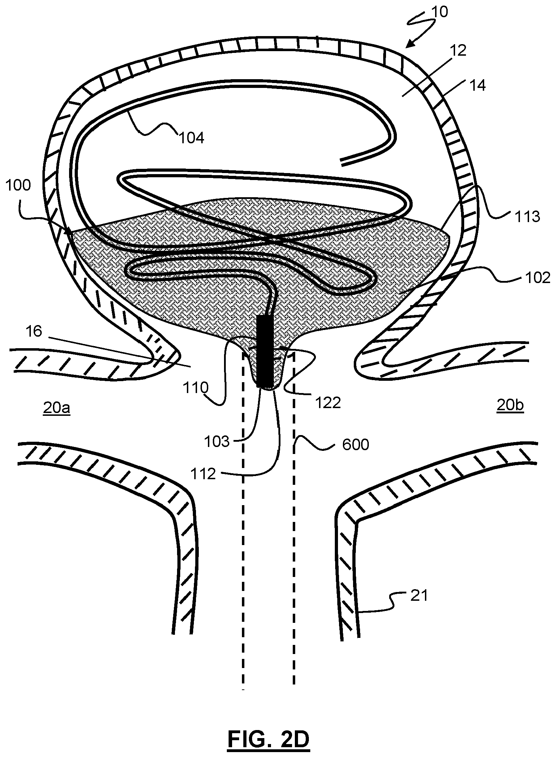

FIG. 2D illustrates the implant 100 during implantation as the occluding portion 102 expands to contact walls 14 within the aneurysm sac 12. The occluding portion 102 can engage the aneurysm walls 14 to secure the implant 100 within the aneurysm and block the neck 16 of the aneurysm. The closure mechanism 122 can be positioned near the center of the aneurysm neck 16. As the occluding portion 102 expands to the walls 14, the occluding portion 102 can begin to form a cup or bowl shape having a ridge and a trough, the open end 113 defining the ridge, and the closure mechanism 122 positioned at the trough.

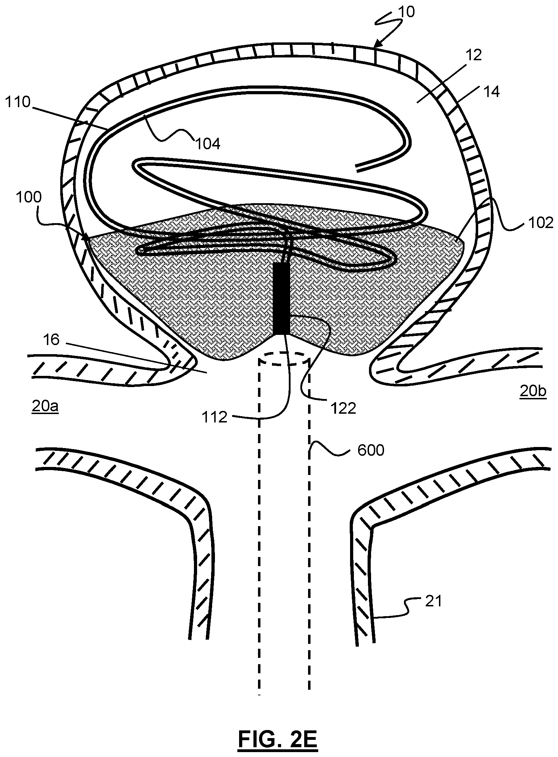

FIG. 2E illustrates the implant 100 in a final implanted configuration such as described in relation to FIG. 1B. The closure mechanism 122 can be moved distally by the delivery system past the plane of the aneurysm 16 and into the aneurysm sac 12 prior to detachment from the delivery system. Additionally, or alternatively, the occluding portion 102 can have a predetermined shape configured to facilitate the movement of the closure mechanism 122 past the plane of the aneurysm neck 16 once the implant 100 is implanted. Once the implant 100 is implanted as illustrated in FIG. 2E, the delivery system can be detached and withdrawn, and the microcatheter 600 can be moved or extracted from the patient.

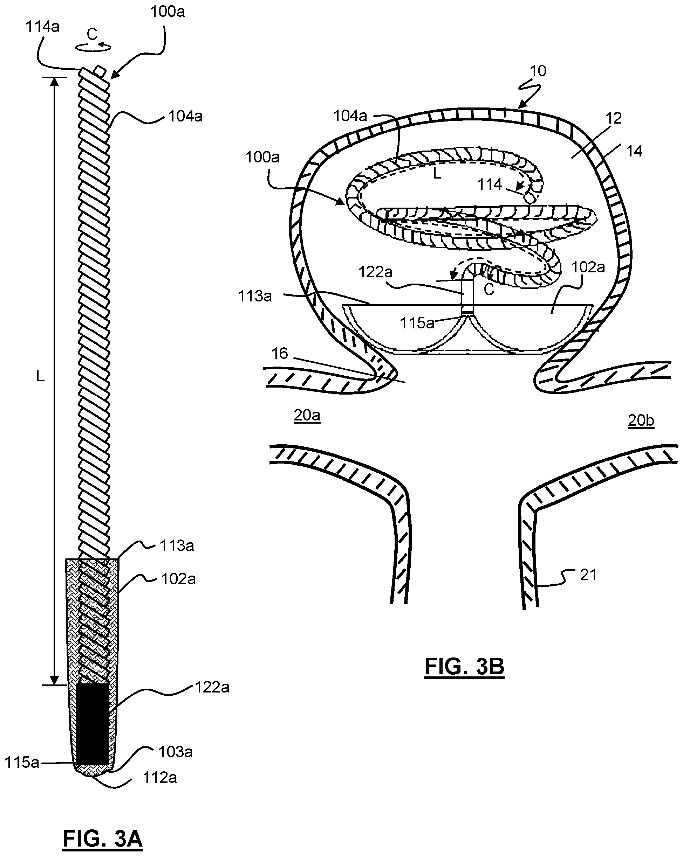

FIG. 3A illustrates an example implant 100a in a collapsed or delivery configuration. In the delivery configuration, the implant 100a can be collapsed to a size that can be delivered to a treatment site through a catheter inserted in vasculature of a patient. FIG. 3B illustrates the example implant 100a illustrated in FIG. 3A in an expanded or implanted configuration. In the implanted configuration, the implant 100a can be positioned in a sac 12 of an aneurysm 10 to divert blood flow from a neck 16 of the aneurysm 10 and fill the sac 12 with embolic material. The embolic material in the aneurysm sac 12 can promote the formation of a thrombotic mass in the aneurysm 10, and the diversion of blood flow from the aneurysm neck 16 can induce venous stasis in the aneurysm and reduce the likelihood that the aneurysm recanalizes after an aneurysm treatment procedure.

Comparing the example implant 100a illustrated in FIGs. 3A and 3B to the implant 100 illustrated in FIGs. 1A through 2E, the elongated portion 104a of the implant 100a in FIGs. 3A and 3B can be an embolic coil 104a rather than a braid 104 as illustrated in FIGs. 1A through 2E. The implant 100a illustrated in FIGs. 3A and 3B can be implanted following a procedure like that illustrated in FIGs. 2A through 2E.

Referring collectively to FIGs. 3A and 3B, the implant 100a can include the embolic coil 104a, an occluding portion 102a, and a closure mechanism 122a. The occluding portion 102a can be braided. Portions of the implant 100a, including the embolic coil 104a, can be made from a memory shape material having a predetermined shape and a deformed shape. The memory shape material can be in the deformed shape when the implant 100a is in the delivery configuration and can move toward the predetermined shape when the implant 100a is in the expanded or implanted configuration. When expanded or implanted, the memory shape material can be restricted by anatomical geometries and the memory shape material can take a shape that approaches the predetermined shape but does not match the predetermined shape due to the anatomical restrictions.

The closure mechanism 122a can join the embolic coil 104a to the occluding portion 102a and can define a boundary between the embolic coil 104a and occluding portion 102a. The closure mechanism 122a can include a band, and/or a mechanism for detachably attaching to a delivery system.

Referring to FIG. 3A, in the delivery configuration, the closure mechanism 122a can be positioned at the proximal end 112a of the implant 100a, the embolic coil 104a can extend distally from the closure mechanism 122a to a distal end 114a of the implant 100a, and the occluding portion 102a can extend proximally from the closure mechanism 122a and include a fold 103a at the proximal end 112a of the implant 100a. In the delivery configuration, the occluding portion 102a can extend distally from the fold 103a to wrap around the closure mechanism 122a and part of the length L of the embolic coil 104a, and the occluding portion 102a can have an open end 113a that circles the embolic coil 104a.

To achieve this folded structure, the occluding portion 102a and the embolic portion 104a can first be connected to the closure mechanism 122a so the embolic coil 104a extends distally from the closure mechanism 122a and the occluding portion 102a extends proximally from the closure mechanism 122a. The occluding portion 102a can then be inverted, and the open end 113a can move distally from a position that is in the proximal direction in relation to the closure mechanism 122a, over the occluding portion 102a, over the closure mechanism 122a, and over part of the embolic coil 104a, thereby forming the fold 103a at the proximal end 112a of the implant 100a and moving the open end 113a to encircle the embolic coil 104a. In the delivery configuration, the closure mechanism 122a can include a detachment feature detachably attached to a delivery system.

Referring to FIG. 3B, in the implanted configuration, the occluding portion 102a can extend across the aneurysm neck 16, the embolic coil 104a can wind within the aneurysm sac 12, and the closure mechanism 122a can be positioned near the center of the aneurysm neck 16. In the implanted configuration, the occluding portion 102a can extend from the closure mechanism 122a to engage a wall 14 and anchor within the aneurysm sac 12. By anchoring within the sac 12, the occluding portion 102a can maintain proper positioning of the implant 100a in the aneurysm 10 and prevent portions of the implant 100a, such as the embolic coil 104a, from extending into a blood vessel 20a, 20b, 21 or exiting the aneurysm sac 12.

The implanted occluding portion 102a can form a cup shape, and the embolic coil 104a can wind or loop around in a complex coiled shape within the cupped occluding portion 102a and within the aneurysm sac 12. The open end 113a of the occluding portion 102a can define a ridge of the cupped shape, and the closure mechanism 122a can be positioned centrally within the cupped shape, or near the trough. The closure mechanism 122a can constrain an end 115a of the occluding portion 102a to a completely closed size or a small enough size to inhibit blood flow from entering the aneurysm neck 16. The looped embolic coil 104a can press against the cupped occluding portion 102a and provide a force against an interior surface of the occluding portion 102a to press an exterior surface of the occluding portion 102a to the aneurysm wall 14 to further secure the anchoring of the cupped occluding portion 102a within the aneurysm sac 12.

The looped embolic coil 104a can fill a majority of the aneurysm sac 12. The implanted occluding portion 102a can be a braided mesh with a porosity sized to inhibit blood flow into the aneurysm 10.

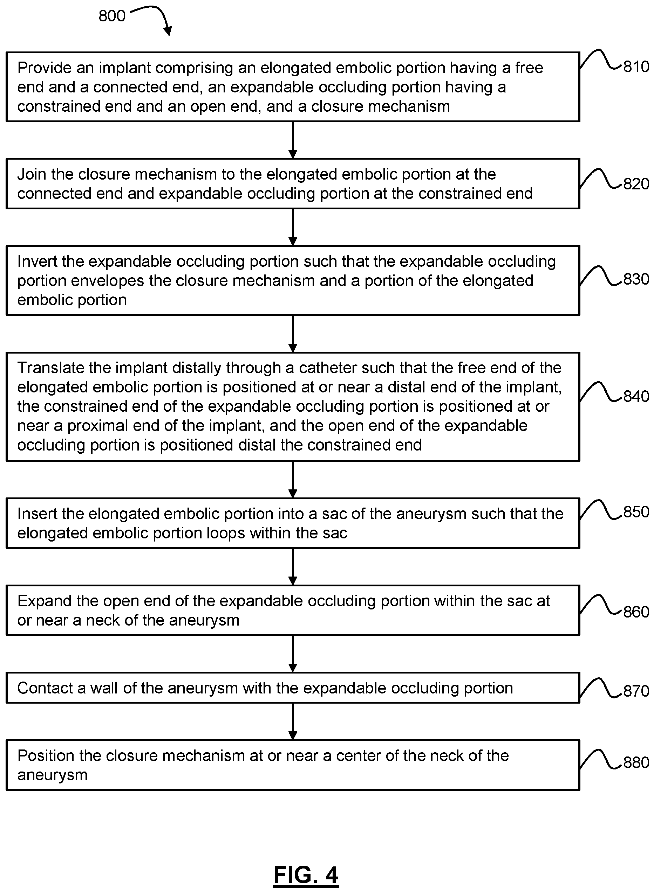

FIG. 4 is a flow diagram outlining example method steps for treating an aneurysm. The method steps can be implemented by example devices presented herein or by other means as would be known to one of ordinary skill in the art. Treating an aneurysm can include steps for assembling or manufacturing an implant and/or delivery system and steps for implanting the implant. Steps 810 through 830 can describe steps for assembling or manufacturing an implant, and steps 840 through 880 can describe steps for implanting the implant, for example.

Referring to method 800 outlined in FIG. 8, in step 810, an implant having an elongated embolic portion with a free end and a connected end, an expandable occluding portion with a constrained end and an open end, and a closure mechanism. In step 820, the closure mechanism can be joined to the elongated embolic portion at the connected end and the expandable occluding portion at the constrained end. In step 830, the expandable occluding portion can be inverted such that the expandable occluding portion envelopes the closure mechanism and a portion of the elongated embolic portion. In step 840, the implant can be translated distally through a catheter such that the free end of the elongated embolic portion is positioned at or near a distal end of the implant, the constrained end of the expandable occluding portion is positioned at or near a proximal end of the implant, and the open end of the expandable occluding portion is positioned distal the constrained end. In step 850, the elongated embolic portion can be inserted into a sac of the aneurysm such that the elongated embolic portion loops within the sac. In step 860, the open end of the expandable occluding portion can be expanded within the sac at or near a neck of the aneurysm. In step 870, a wall of the aneurysm can be contacted with the expandable occluding portion. In step 880, the closure mechanism can be positioned at or near a center of the aneurysm neck.

The descriptions contained herein are examples of embodiments of the invention and are not intended in any way to limit the scope of the invention. As described herein, the invention contemplates many variations and modifications of an implant, system, or method that can be used to occlude and fill an aneurysm. Variations can include but are not limited to alternatively geometries of elements and components described herein, utilizing alternative materials for each component or element (e.g. radiopaque materials, memory shape materials, etc.), utilizing additional components including components to deliver the implant to a treatment site, position the implant at a treatment site, retract the implant, and/or eject a portion of the implant from a catheter, utilizing additional component to perform functions describe herein, and utilizing additional components to perform functions not described herein, for example. These modifications would be apparent to those having ordinary skill in the art to which this invention relates and are intended to be within the scope of the claims which follow.

### Embodiments

1. A method of treating an aneurysm, the method comprising:
   providing an implant comprising an elongated embolic portion comprising a free end and a connected end, an expandable occluding portion comprising a constrained end and an open end, and a closure mechanism;
   joining the closure mechanism to the elongated embolic portion at the connected end and expandable occluding portion at the constrained end;
   inverting the expandable occluding portion such that the expandable occluding portion envelopes the closure mechanism and a portion of the elongated embolic portion;
   translating the implant distally through a catheter such that the free end of the elongated embolic portion is position approximate a distal end of the implant, the constrained end of the expandable occluding portion is positioned approximate a proximal end of the implant, and the open end of the expandable occluding portion is positioned distal the constrained end;
   inserting the elongated embolic portion into a sac of the aneurysm such that the elongated embolic portion loops within the sac;
   expanding the open end of the expandable occluding portion within the sac approximate a neck of the aneurysm;
   contacting a wall of the aneurysm with the expandable occluding portion; and
   positioning the closure mechanism approximate a center of the neck of the aneurysm.
2. The method of embodiment 1 further comprising:
   forming the elongated embolic portion and the expandable occluding portion from a contiguous tubular braid.
3. The method of embodiment 1 further comprising:
   forming the elongated embolic portion from an embolic coil; and
   forming the expandable occluding portion from a tubular braid.
4. The method of embodiment 1 further comprising:
   forming the expandable occluding portion into a bowl shape comprising a ridge and a trough, the ridge defined by the open end of the expandable occluding portion and positioned approximate the wall of the aneurysm and the trough centered around the closure mechanism.
5. The method of embodiment 1 further comprising:
   forming the expandable occluding portion from a braided mesh; and
   sizing pores of the braided mesh once expanded to inhibit blood flow into the aneurysm.
6. The method of embodiment 1 further comprising:
   anchoring the expandable occluding portion to the aneurysm wall; and
   inhibiting the elongated embolic portion from exiting the aneurysm sac.
7. The method of embodiment 6 wherein anchoring the expandable occluding portion further comprises providing a force from the elongated embolic portion against an interior surface of the expandable occluding portion to press an exterior surface of the expandable occluding portion to the aneurysm wall.

## Claims

1. An implant comprising:
an embolic portion;
an occluding portion; and
a closure mechanism in communication with, and defining a boundary between, the embolic portion and the occluding portion,
wherein the implant is movable from a delivery configuration sized to traverse through a lumen of a catheter to an implanted configuration sized to secure within an aneurysm,
wherein in the delivery configuration, the embolic portion extends proximally from a distal end of the implant to the closure mechanism and the occluding portion extends proximally from the closure mechanism, comprises a fold approximate a proximal end of the implant, extends distally to encompass the closure mechanism and a portion of the embolic portion, and comprises an open end encircling the embolic portion, and
wherein in the implanted configuration, the embolic portion is sized to wind within a sac of the aneurysm and fill a majority of the sac and the occluding portion is sized to contact a wall of the aneurysm and occlude at least a portion of a neck of the aneurysm.

2. The implant of claim 1 wherein the embolic portion and the occluding portion are braided tubular portions.

3. The implant of claim 1 or claim 2 wherein the embolic portion comprises an embolic coil, and wherein the occluding portion comprises a tubular braid.

4. The implant of any one of the preceding claims wherein, in the implanted configuration, the occluding portion forms a cup shape such that the open end defines a ridge of the cup shape, and the closure mechanism is positioned approximate a trough of the cup shape.

5. The implant of any one of the preceding claims wherein, in the implanted configuration, the occluding portion comprises a braided mesh comprising pores sized to inhibit blood flow into the aneurysm.

6. The implant of any one of the preceding claims wherein, in the implanted configuration, the occluding portion is configured to anchor to the aneurysm wall and inhibit the embolic portion from exiting the aneurysm sac.

7. The implant of any one of the preceding claims wherein, in the implanted configuration, the embolic portion comprises a length measurable from the distal end of the implant to the closure mechanism and a substantially uniform circumference along a majority of the length.

8. An implant suitable for endovascular treatment of an aneurysm in a patient, the implant comprising:
a substantially tubular structure comprising a constrained proximal end region and an open distal end region, the tubular structure movable from a collapsed condition to an expanded condition, comprising dimensions in the collapsed condition suitable for insertion through vasculature of the patient and through a neck of the aneurysm, and comprising a shape in the expanded condition capable of contacting an interior wall of the aneurysm approximate the open distal end region and converging approximate a center of the neck of the aneurysm approximate the constrained proximal end region;
an elongated embolic member attached to the constrained proximal end region of the tubular structure and extending distally therefrom, the elongated embolic member positioned to be encircled by the tubular structure when the tubular structure is in the collapsed condition and bendable to loop within a sac of the aneurysm; and
a band disposed on the constrained proximal end region of the tubular structure, the band inhibiting expansion of the constrained proximal end region.

9. The implant of claim 8 wherein the elongated embolic member and the tubular structure are braided tubular portions.

10. The implant of claim 8 or claim 9 wherein the elongated embolic member comprises an embolic coil, and wherein the tubular structure comprises a braid.

11. The implant of any one of claims 8 to 10 wherein the shape of the tubular structure in the expanded condition comprises a ridge defined by the open distal end region and a trough centered around the constrained proximal end region.

12. The implant of any one of claims 8 to 11 wherein tubular structure comprises a braided mesh and wherein, in the expanded condition, the braided mesh comprises pores sized to inhibit blood flow into the aneurysm.

13. The implant of any one of claims 8 to 12 wherein, in the expanded configuration, the tubular structure is sized to anchor to the aneurysm wall and inhibit the elongated embolic member from exiting the aneurysm sac.
